# EUROPEAN PATENT APPLICATION

(11) **EP 4 772 248 A2**
(43) Date of publication of application: **08.07.2026**
(21) Application number: 26180001.5
(22) Date of filing: 09.10.2023
(51) Int. Cl.: A61P 35/00

(54) **USE OF C/EBP-BETA ANTAGONIST AND IMMUNOMODULATOR**

(30) Priority: 07.10.2022 US 202263414397 P
(62) Divisional of application: 23875894.0
(71) Applicant: Sapience Therapeutics, Inc., Tarrytown, NY 10591 (US)
(72) Inventor: KAPPEL, Barry, Jay, Tarrytown, NY, 10591 (US); ROTOLO, Jimmy, Andrew, Tarrytown, NY, 10591 (US); SCUOPPO, Claudio, Tarrytown, NY, 10591 (US)
(74) Representative: Vossius & Partner Patentanwälte Rechtsanwälte mbB

(57) **Abstract**

Provided are methods of administering a peptide antagonist of CCAAT/enhancer-binding protein beta (C/EBP?) in combination with an immunomodulating agent.

## Description

### CROSS-REFERENCE TO RELATED APPLICATION

This application claims the benefit of priority of US provisional application Serial No. 63/414,397, filed on October 7, 2022.

### REFERENCE TO AN ELECTRONIC SEQUENCE LISTING

The contents of the electronic sequence listing (File name: Sapience020WO1.xml; Size: 4,534 bytes; and Date of Creation: October 6, 2023) is herein incorporated by reference in its entirety.

### BACKGROUND

CCAAT/Enhancer Binding Protein β (C/EBPβ) is a basic leucine zipper (bZIP) transcription factor that causes aberrant gene activation in many cancers. Upregulated or overactivated C/EBPβ drives oncogenesis by promoting tumor survival and proliferation, and is a critical regulator of the immunosuppressive environment (Homma 2006; Ruffell 2009). Specifically, C/EBPβ regulates macrophage differentiation, promoting the expression of M2 myeloid-derived suppressor cells (MDSCs) that contribute to suppression of antitumor immunity and correlate with poor prognosis (Marigo 2010). Reprogramming tumor-associated macrophages (TAMs) from M2 to M1 phenotype represents a potential strategy to enhance antitumor immunity.

Immune-checkpoint inhibitors (ICI) have shown unprecedented success in immunogenic tumors such as melanoma, however many patients with advanced solid tumors fail to respond well or at all to ICI therapy. Refractory tumors are commonly classified as "cold" and demonstrate either a lack of infiltration of T cells into the tumor stroma or an immunosuppressive environment that excludes both T cell infiltration and activity. As an immune-suppressive tumor microenvironment (TME) is a major hurdle to the success of ICI therapy in solid cancers, the ability to convert the TME to an immune-active state represents great promise for improving responses.

ST101 is a novel peptide antagonist that prevents C/EBPβ dimerization and inhibits C/EBPβ-dependent gene expression. Confirmed responses in melanoma and other tumors prompted evaluation of ST101 impact on macrophage differentiation.

### SUMMARY OF THE INVENTION

Some of the main aspects of the present invention are summarized below. Additional aspects are described in the Detailed Description of the Invention, Examples, Drawings, and Claims sections of this disclosure. The description in each section of this disclosure is intended to be read in conjunction with the other sections. Furthermore, the various embodiments described in each section of this disclosure can be combined in various different ways, and all such combinations are intended to fall within the scope of the present invention.

We demonstrate that ST101 exposure reprograms M2-like macrophages to the immune-promoting M1-like phenotype, both *in vitro* and *in vivo.* Macrophage repolarization results in restoration of cytotoxic T cell activity and cooperates with anti-PD-1 therapy in a triple negative breast cancer model *in vivo* to enhance anti-tumor responses. These results identify ST 101 as a novel approach to enhance macrophage anti-tumor activity and support its utility for combination strategies in cancers with poor response to ICIs.

Accordingly, the disclosure provides a combination approach for administering a peptide antagonist of CCAAT-enhancer-binding protein β (C/EBPβ) and an immunomodulator. In one embodiment, a method is provided for inhibiting growth of a solid tumor in a subject, the method comprising combination therapy with: (i) a pharmaceutical composition comprising an effective amount of a peptide antagonist of C/EBPβ and (ii) a pharmaceutical composition comprising an effective amount of an immunomodulator; wherein the pharmaceutical composition comprising the antagonist of C/EBPβ and the pharmaceutical composition comprising the immunomodulator are administered to the subject together or separately.

Another embodiment is a method of reducing solid tumor volume in a subject, the method comprising combination therapy with: (i) a pharmaceutical composition comprising an effective amount of a peptide antagonist of C/EBPβ and (ii) a pharmaceutical composition comprising an effective amount of an immunomodulator; wherein the pharmaceutical composition comprising the antagonist of C/EBPβ and the pharmaceutical composition comprising the immunomodulator are administered to the subject together or separately.

An additional embodiment provides a method of treating a solid tumor in a in a subject, the method comprising combination therapy with: (i) a pharmaceutical composition comprising an effective amount of a peptide antagonist of C/EBPβ and (ii) a pharmaceutical composition comprising an effective amount of an immunomodulator: wherein the pharmaceutical composition comprising the antagonist of C/EBPβ and the pharmaceutical composition comprising the immunomodulator are administered to the subject together or separately.

Also provided is a pharmaceutical composition comprising an effective amount of a peptide antagonist of C/EBPβ and a pharmaceutical composition comprising an effective amount of an immunomodulator for use in a method of combination therapy for inhibiting growth of a solid tumor in a subject, for reducing solid tumor volume in a subject, and/or for treating a solid tumor in a human subject.

In a preferred embodiment, the antagonist of C/EBPβ is ST101, and the immunomodulator is a PD-1 inhibitor.

In some embodiments, the solid tumor is a melanoma, a carcinoma, or a sarcoma. In particular embodiments, the subject has been diagnosed with locally advanced or metastatic breast cancer (LA/MBC), melanoma, glioblastoma (GBM), or castration-resistant prostate cancer (CRPC).

In one embodiment, the peptide antagonist comprises the D-amino acid sequence VAEAREELERLEARLGQARGEL (SEQ ID NO: 1). In another embodiment, the peptide antagonist comprises the amino acid sequence LEGRAQGLRAELRELEERAEAV (SEQ ID NO: 3). In some embodiments, the peptide antagonist is a cell-penetrating peptide. For example, the peptide antagonist can comprise a cell-penetrating sequence. In a particular embodiment, the peptide antagonist is ST101 (SEQ ID NO: 2).

In some embodiments, the peptide antagonist is administered to the subject at a dose of about 0.5-16 mg/kg. In one embodiment, the dose of ST101 is about 500 mg.

In a certain aspect of the invention, the immunomodulator is an antibody or an antibody-drug conjugate, for example, selected from the group consisting of amivantamab, belantamab mafodotin-blmf, bevacizumab, cetuximab, denosumab, dinutuximab, enfortumab vedotin-ejfv, margetuximab, naxitamab-gqgk, necitumumab, panitumumab, pertuzumab, ramucirumab, Sacituzumab govitecan-hziy, tebentafusp-tebn, tisotumab vedotin, trastuzumab, trastuzumab deruxtecan, and trastuzumab emtansine. In a particular aspect, the antibody is an anti-PD-1 antibody.

In one aspect of the invention, the immunomodulator is selected from the group consisting of a checkpoint inhibitor, a cytokine, an immune adjuvant. The checkpoint inhibitor can target, for example, at least one of PD-1, PD-L1, CTLA-4, or LAG-3. In particular embodiments, the checkpoint inhibitor is atezolizumab, avelumab, cemiplimab, dostarlimab, durvalumab, ipilimumab, nivolumab, pembrolizumab, relatimab, or a combination thereof.

In one embodiment, the immunomodulator is a cytokine that targets, for instance, at least one of the IL-2 pathway, the IL-2R pathway, the IFNAR1 pathway, or the IFNAR2 pathway. Examples of cytokines for use in the methods of the invention include aldesleukin, granulocyte-macrophage colony-stimulating factor, interferon alfa-2a, interferon alfa-2b, and peginterferon alfa-2b.

The immunomodulator can be an immune adjuvant. In one embodiment, the immune adjuvant targets the Toll-like receptor 7 pathway or the Toll-like receptor 3 pathway. Particular examples of immune adjuvants include Imiquimod and Poly ICLC.

In one embodiment, the pharmaceutical composition comprising the antagonist of C/EBPβ and/or the pharmaceutical composition comprising the immunomodulator is administered parenterally, for example, intravenously.

In some embodiments, the pharmaceutical composition comprising the antagonist of C/EBPβ and the pharmaceutical composition comprising the immunomodulator are administered to the subject on different days.

In certain embodiments, the pharmaceutical composition comprising the antagonist of C/EBPβ is administered once weekly for at least three weeks, or once every two weeks for at least four weeks. In certain embodiments, the pharmaceutical composition comprising the immunomodulator is administered once weekly for at least three weeks, or once every two weeks for at least four weeks, or once every three weeks for at least six weeks.

### BRIEF DESCRIPTION OF THE DRAWINGS

**FIG. 1** shows a schematic diagram of the protocol for the human peripheral blood mononuclear cells (hPBMCs) M1/M2 polarization model described in Example 1.
**FIG. 2A-2B** show that ST101 shifts the M2 program to an M1-like phenotype in cultured hPBMCs derived from Donor 1 **(****FIG. 2A****)** and Donor 2 **(****FIG. 2B****).** Cells were treated with the indicated concentrations of ST101 and expression of CD163 and CD68 was measured by flow cytometry.
**FIG. 3A-3D** show that ST101 substantially reduces hPBMC-derived M2 cells in cultures derived from Donor 1 **(****FIG. 3A-3B****)** and Donor 2 **(****FIG. 3C-3D****).** Cells were treated with the indicated concentrations of ST101 and expression of CD163 and CD68 was measured by flow cytometry **(****FIG. 3A****,** **3C****).** **FIG. 3B** and **3D** show the ratio of M2 cells to M1 cells after treatment with the indicated concentrations of ST101.
**FIG. 4A-4B** show that ST101 inhibits M2 macrophage polarization in hPBMCs derived from Donor 1. Cells were treated with the indicated concentrations of ST101 and expression of CD163 **(****FIG. 4A****)** and CD68 **(****FIG. 4B****)** was measured by flow cytometry
**FIG. 5A-5C** show that ST101 impacts the tumor microenvironment. Nanostring analysis of tumor samples from human patients treated with ST101 shows decreased IL6 signaling **(****FIG. 5A****),** increased tumor-infiltrating macrophages **(****FIG. 5B**). and decreased regulatory T cells **(****FIG. 5C****).**
**FIG. 6** shows that ST101 restores activation of cultured human CD8+ T cells co-incubated with immunosuppressive M2 macrophages.
**FIG. 7** shows enhanced tumor grown inhibition (TGI) by treatment with a combination of a C/EBPβ antagonist (ST101) and an immune checkpoint inhibitor (anti-PD-1 antibody).

### DETAILED DESCRIPTION OF THE INVENTION

In order that the present invention can be more readily understood, certain terms are first defined. Additional definitions are set forth throughout the disclosure. Unless defined otherwise, all technical and scientific terms used herein have the same meaning as commonly understood by one of ordinary skill in the art to which this invention is related.

Any headings provided herein are not limitations of the various aspects or embodiments of the invention, which can be had by reference to the specification as a whole. Accordingly, the terms defined immediately below are more fully defined by reference to the specification in its entirety.

All references cited in this disclosure are hereby incorporated by reference in their entireties. In addition, any manufacturers' instructions or catalogues for any products cited or mentioned herein are incorporated by reference. Documents incorporated by reference into this text, or any teachings therein, can be used in the practice of the present invention. Documents incorporated by reference into this text are not admitted to be prior art.

### I. Definitions

The phraseology or terminology in this disclosure is for the purpose of description and not of limitation, such that the terminology or phraseology of the present specification is to be interpreted by the skilled artisan in light of the teachings and guidance.

As used in this specification and the appended claims, the singular forms "a," "an," and "the" include plural referents, unless the context clearly dictates otherwise. The terms "a" (or "an") as well as the terms "one or more" and "at least one" can be used interchangeably.

Furthermore, "and/or" is to be taken as specific disclosure of each of the two specified features or components with or without the other. Thus, the term "and/or" as used in a phrase such as "A and/or B" is intended to include A and B, A or B, A (alone), and B (alone). Likewise, the term "and/or" as used in a phrase such as "A, B, and/or C" is intended to include A, B, and C: A, B, or C: A or B; A or C; B or C; A and B: A and C; B and C; A (alone); B (alone); and C (alone).

Wherever embodiments are described with the language "comprising," otherwise analogous embodiments described in terms of "consisting of" and/or "consisting essentially of" are included.

Units, prefixes, and symbols are denoted in their Système International d'Unités (SI) accepted form. Numeric ranges are inclusive of the numbers defining the range, and any individual value provided herein can serve as an endpoint for a range that includes other individual values provided herein. For example, a set of values such as 1, 2, 3, 8, 9, and 10 is also a disclosure of a range of numbers from 1-10, from 1-8, from 3-9, and so forth. Likewise, a disclosed range is a disclosure of each individual value (*i.e.,* intermediate) encompassed by the range, including integers and fractions. For example, a stated range of 5-10 is also a disclosure of 5, 6, 7, 8, 9, and 10 individually, and of 5.2, 7.5, 8.7, and so forth.

Unless otherwise indicated, the terms "at least" or "about" preceding a series of elements is to be understood to refer to every element in the series. The term "about" preceding a numerical value includes ± 10% of the recited value. For example, a concentration of about 1 mg/mL includes 0.9 mg/mL to 1.1 mg/mL. Likewise, a concentration range of about 1% to 10% (w/v) includes 0.9% (w/v) to 11% (w/v).

The terms "polypeptide," "peptide," and "protein" are used interchangeably to refer to polymers of amino acids of any length, and their salts. The polymer can be linear or branched, can comprise modified amino acids, and can be interrupted by non-amino acids. Except where indicated otherwise, e.g., for the abbreviations for the uncommon or unnatural amino acids set forth herein, the three-letter and one-letter abbreviations, as used in the art, are used herein to represent amino acid residues. Except when preceded with a "D" or in lower case, the amino acid is an L-amino acid. Groups or strings of amino acid abbreviations are used to represent peptides. Except where specifically indicated, peptides are indicated with the N-terminus of the left and the sequence is written from the N-terminus to the C-terminus.

A "retro inverso" peptide has a reversed amino acid sequence, relative to a reference L-amino acid sequence, and is made up of all D-amino acids (inverting the α-center chirality of the amino acid subunits) to help maintain side-chain topology similar to that of the original L-amino acid peptide.

An "isolated" molecule is one that is in a form not found in nature, including those which have been purified.

An "active agent" is an ingredient that is intended to furnish biological activity. The active agent can be in association with one or more other ingredients. An active agent that is a peptide can also be referred to as an "active peptide."

An "effective amount" of an active agent is an amount sufficient to carry out a specifically stated purpose.

The term "pharmaceutical composition" refers to a preparation that is in such form as to permit the biological activity of the active ingredient to be effective and which contains no additional components that are unacceptably toxic to a subject to which the composition would be administered. Such composition can be sterile and can comprise a pharmaceutically acceptable carrier, such as physiological saline. Suitable pharmaceutical compositions can comprise one or more of a buffer (e.g., acetate, phosphate, or citrate buffer), a surfactant (*e.g.,* polysorbate), a stabilizing agent (*e.g.,* polyol or amino acid), a preservative (e.g., sodium benzoate), and/or other conventional solubilizing or dispersing agents.

A "subject" or "individual" or "animal" or "patient" or "mammal," is any subject, particularly a mammalian subject, for whom diagnosis, prognosis, or therapy is desired. Mammalian subjects include humans, domestic animals, farm animals, sports animals, and laboratory animals including, e.g., humans, non-human primates, canines, felines, porcines, bovines, equines, rodents, including rats and mice, rabbits, etc.

A "control patient" is a subject that has not received a treatment of the invention. A "control population" or a "population of control patients" is a group of subjects that have not received a treatment of the invention. A control patient or subject in the control population has the same disease or disorder as the subject being compared to the control patient or control population. For example, a clinical outcome of a cancer patient receiving the compositions or method of the invention is compared with the average (median) outcome of subjects having the same type of cancer who did not receive a pharmaceutical composition or method of the invention. In some embodiments, the control patient or patients in the control population have received a treatment other than a treatment of the invention, for example, a standard-of-care treatment.

Terms such as "treating" or "treatment" or "to treat" or "alleviating" or "to alleviate" refer to therapeutic measures that cure, slow down, lessen symptoms of, and/or halt progression of a diagnosed pathologic condition or disorder. In certain embodiments, a subject is successfully "treated" for a disease or disorder if the patient shows total, partial, or transient alleviation or elimination of at least one symptom or measurable physical parameter associated with the disease or disorder.

An "antagonist" is a substance that prevents, blocks, inhibits, neutralizes, or reduces a biological activity or effect of another molecule, such as a receptor or ligand.

The terms "inhibit," "block," and "suppress" are used interchangeably and refer to any statistically significant decrease in occurrence or activity, including full blocking of the occurrence or activity. For example, "inhibition" can refer to a decrease of about 10%, 20%, 30%, 40%, 50%, 60%, 70%, 80%, 90% or 100% in activity or occurrence. An "inhibitor" is a molecule, factor, or substance that produces a statistically significant decrease in the occurrence or activity of a process, pathway, or molecule.

A "tumor" or "solid tumor" is a mass of neoplastic cells, such as cancer cells. The terms "advanced," "metastatic," and "advanced/metastatic" are used interchangeably to describe a cancer in which malignant cells have migrated from the original tumor to another location, for example, another organ, in a patient's body.

A "neoplastic cell" or "neoplasm" typically has undergone some form of mutation/transformation, resulting in abnormal growth as compared to normal cells or tissue of the same type. Neoplasms include morphological irregularities, as well as pathologic proliferation. Neoplastic cells can be benign or malignant. Malignant neoplasms, *i.e.,* cancers, are distinguished from benign in that they demonstrate loss of differentiation and orientation of cells, and have the properties of invasion and metastasis.

### II. Peptides and Compositions

### C/EBPβ

C/EBPβ represents a prime target for development of peptide anatagonists, due to its reliance on basic leucine zipper (bZIP) interactions with co-factors. In order to associate with DNA and transactivate gene expressions, C/EBPβ dimerizes with binding partners via interactions between their bZIP domains. In addition to homodimerization, C/EBPβ forms heterodimers with bZIP-containing transcription factors such as Jun/Fos, C/EBPγ (Huggins 2013), delta-interacting protein A (Bezy 2005), and the CREB/ATF family (Zhao 2014).

Activating transcription factor 5 (ATF5) is a CREB/ATF factor that has been determined to associate with and activate C/EBPβ in HEK293 and HCT116 cancer cells, resulting in transactivation of a pro-survival phenotype (Zhang 2015). ATF5 is highly expressed in many cancers, including glioma, where it contributes to the oncogenic phenotype by driving the overexpression of Bcl-2 family proteins and survivin, but is largely not found in differentiated cell types (Sheng 2010). Overexpression of the truncated bZIP domain of ATF5 lacking a DNA-binding domain in glioma and other tumor cells resulted in cancer cell cytotoxicity (Angelastro 2006): administration of a peptide containing the truncated bZIP domain produced similar results (Cates 2016; Karpel-Massler 2016).

### C/EBPβ Antagonist Peptides

In some embodiments, methods of the invention comprise treating a patient having a solid tumor with combination therapy comprising an effective amount of a peptide antagonist of C/EBPβ and an immunomodulator. In one embodiment, the peptide antagonist of C/EBPβ comprises the D-amino acid sequence VAEAREELERLEARLGQARGEL (SEQ ID NO: 1), which is a retro inverso variant of the wild-type ATF5 bZIP domain. Peptide antagonists of C/EBPβ can be designed, for example, as described in Example 1 of WO 2021/262604.

The peptide antagonist of C/EBPβ can be a cell-penetrating peptide. In one embodiment, the peptide comprises a cell-penetrating domain. Numerous cell-penetrating peptide sequences are described and characterized in the literature *(see* WO 2019/136125). In one embodiment, the peptide is a cyclic peptide. Cyclized peptides, for example, using hydrocarbon staples (Bernal 2007; Bird 2016) or other cyclization methods known in the art, can enter cells via passive diffusion, endocytosis/endosomal escape, or other mechanisms (Dougherty 2019). Peptides can also be delivered to cells via mechanisms that exploit cellular receptors, for example, integrin-targeting, RGD-like sequences. Alternatively, peptides can be encapsulated and delivered to cells in vesicles, such as exosomes or liposomes, or in micelles.

The ability of a peptide based on the native ATF5 bZIP domain to antagonize the activity of C/EBPβ can be measured by methods described herein, for instance, in Example 2 of WO 2021/262604. The cytotoxic activity of a peptide antagonist of C/EBPβ can be measured *in vitro* by known assays and/or *in vivo* using known tumor models; for example, WO 2019/136125 describes such assays and models.

ST101 is an all D-amino acid peptide that displays potent anti-tumor activity *in vitro* and *in vivo* and resistance to proteolytic degradation. In particular, we have previously demonstrated cytotoxicity of ST101 in HL60 (promyelocytic leukemia), AML14 (acute myeloid leukemia), SET2 (megakaryoblastic leukemia), A375 (melanoma), MCF7 (breast carcinoma), U87 (glioblastoma), U251 (glioblastoma), DU145 (prostate cancer), A549 (lung cancer), peripheral blood mononuclear cells (PBMC), and bone marrow mononuclear cells (BMMC) *(see* WO 2019/136125). In addition, subcutaneous administration of ST101 in xenograft mouse model using A375, HL60, MCF7, and U251 cells resulted in significant reduction in tumor volume *(see* WO 2019/136125).

ST101 is comprised of modified domains based on the ATF5 bZIP domain and on the Antennapedia penetratin domain, to allow for cell penetration. The D-amino acid sequence of ST101 is VAEAREELERLEARLGQARGEL*KKWKMRRNQFWLKLOR* (SEQ ID NO: 2), with the cell-penetrating region italicized. ST101 promotes cytotoxic activity in tumor cells by disrupting the association of C/EBPβ with anti-apoptotic transcription factors *(see* WO 2021/262604).

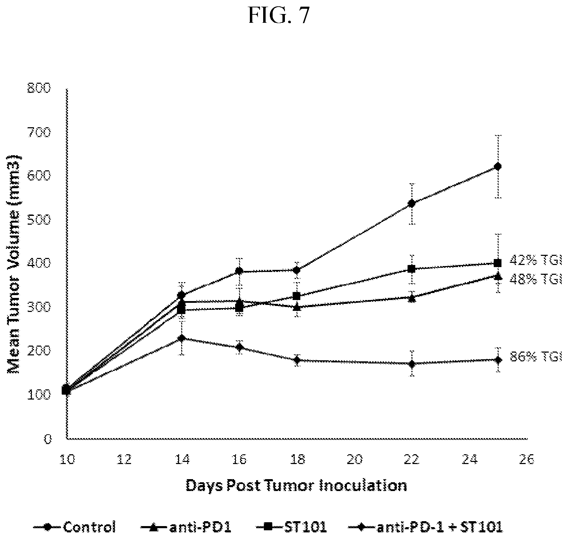

We demonstrate for the first time that ST101 alters the tumor microenvironment and reprograms macrophage differentiation to an M1 phenotype. The potential effect of ST101 on antitumor immunity led us to test whether the combination of a C/EBPβ antagonist, such as ST101, and an immunomodulator, such as an immune checkpoint inhibitor, would result in enhanced anti-tumor activity. The combination of ST101 and PD-1 unexpectedly reduced tumor volume in a mouse model of triple-negative breast cancer, demonstrating approximately 1.8- to 2.0-fold improved activity over either active agent alone. (*See* Example 3; FIG. 7).

### IV. Methods of Preparing C/EBPβ Antagonists

Peptide antagonists of C/EBPβ can be chemically synthesized, for example, using solid-phase peptide synthesis or solution-phase peptide synthesis, or a combination of both. Synthesis may optionally occur as fragments of the peptide that are subsequently combined either chemically or enzymatically.

Alternatively, peptide antagonists of C/EBPβ can be expressed using recombinant methods. For example, nucleic acid molecules encoding ST101 can be constructed by chemical synthesis using an oligonucleotide synthesizer. Nucleic acid molecules can be designed based on the amino acid sequence of ST101 and selection of those codons that are favored in the host cell in which the recombinant ST101 will be produced. Standard methods can be applied to synthesize a nucleic acid molecule encoding a peptide antagonist of C/EBPβ, such as ST101.

Once prepared, the nucleic acid encoding the peptide can be inserted into an expression vector and operably linked to an expression control sequence appropriate for expression of the peptide in a desired host. In order to obtain high expression levels of the peptide, the nucleic acid can be operably linked to or associated with transcriptional and translational expression control sequences that are functional in the chosen expression host.

A wide variety of expression host/vector combinations can be employed to anyone known in the art. Useful expression vectors for eukaryotic hosts include, for example, vectors comprising expression control sequences from SV40, bovine papilloma virus, adenovirus, and cytomegalovirus. Useful expression vectors for bacterial hosts include known bacterial plasmids, such as plasmids from *E. coli,* including pCR1, pBR322, pMB9 and their derivatives, wider host range plasmids, such as M13, and filamentous single-stranded DNA phages.

Suitable host cells include prokaryotes, yeast, insect, or higher eukaryotic cells under the control of appropriate promoters. Prokaryotes include gram negative or gram positive organisms, for example *E. coli* or bacilli. Higher eukaryotic cells can be established or cell lines of mammalian origin, examples of which include Pichia pastoris, 293 cells, COS-7 cells, L cells, C127 cells, 3T3 cells, Chinese hamster ovary (CHO) cells, HeLa cells, and BHK cells. Cell-free translation systems can also be employed.

Peptides can be purified using methods that include, for example, reverse-phase high-performance liquid chromatography (RP-HPLC), multicolumn countercurrent solvent gradient purification (MCSGP), and ion-exchange chromatography.

### Immunomodulators

The methods of the invention involve combination therapy comprising administration of a C/EBPβ antagonist and an immunomodulator. The terms "immunomodulator," "immunomodulating agent," and "immunotherapeutic agent" are interchangeable and refer to an active agent that stimulates or suppresses the immune system of a subject to fight a disease or infection. In the context of the present invention, the disease is a neoplasia or cancer.

Immunomodulators include, for example, immune checkpoint inhibitors; cytokines: targeted antibodies and drug-antibody conjugates; adjuvants, such as imiquimod and polyinosinic-polycytidylic acid (poly ICLC); oncolytic viruses, such as T-VEC: and small molecule drugs, such as thalidomide, lenalidomide, or pomalidomide.

Immune checkpoint inhibitors target immune checkpoint proteins or their ligand(s). Immune checkpoint proteins include, but are not limited to, cytotoxic T lymphocyte-associated antigen 4 (CTLA-4), also known as CD152, programmed cell death protein 1 (PD-1), also known as CD279, lymphocyte-activation gene 3 (LAG-3), also known as CD223, T cell immunoglobulin mucin (TIM-3), also known as HAVcr2, and T cell immunoreceptor with Ig and ITIM domains (TIGIT). Examples of immune checkpoint inhibitors include atezolizumab, avelumab, cemiplimab, dostarlimab, durvalumab, ipilimumab, nivolumab, pembrolizumab, and relatlimab.

Some cytokines, such as interferons, can disrupt the division of cancer cells and slow tumor growth. Other cytokines, such as interleukins (IL), stimulate immune cell growth and proliferation. Examples of immunomodulating cytokines include aldesleukin, granulocyte-macrophage colony-stimulating factor, interferon alfa-2a, interferon alfa-2b, and peginterferon alfa-2b.

Targeted antibodies can be customized to target antigens on the surface of cancer cell, in order to disrupt cancerous activity, particularly unrestrained growth. Some targeted antibodies are conjugated to anti-cancer drugs. Other targeted antibodies are bi-specific, for example, binding both cancer cells and T cells in order to enhance the anti-cancer immune response. Examples of targeted antibodies and drug-antibody conjugates include amivantamab, belantamab mafodotin-blmf, bevacizumab, cetuximab, denosumab, dinutuximab, enfortumab vedotin-ejfv, margetuximab, naxitamab-gqgk, necitumumab, panitumumab, pertuzumab, ramucirumab, Sacituzumab govitecan-hziy, tebentafusp-tebn, tisotumab vedotin, trastuzumab, trastuzumab deruxtecan, and trastuzumab emtansine.

### Compositions and Administration

In certain aspects, the invention provides a combination approach comprising administration of a composition, e.g., a pharmaceutical composition, comprising an effective amount of a peptide antagonist of C/EBPβ, such as ST101, and administration of composition, e.g., a pharmaceutical composition, comprising an effective amount of an immunomodulating agent, such as a checkpoint inhibitor. Methods of administering peptide antagonists of C/EBPβ are described, for example, in WO 2021/262604.

Because the combination therapy of the invention demonstrates synergism between the C/EBPβ antagonist and the immunomodulator, the effective amount of each active agent can, in some embodiments, be lower than the effective amount when each active agent is administered individually. For example, the effective amount of each of the peptide antagonist of C/EBPβ and the immunomodulating agent may be a sub-therapeutic dose.

The compositions are preferably administered parenterally. Parenteral routes of administration include intravenous (IV), intramuscular, intraperitoneal, intrathecal, and subcutaneous. In certain embodiments the composition comprising the peptide antagonist of C/EBPβ and/or the composition comprising the immunomodulator are/is administered to the subject by intravenous infusion.

The composition comprising a peptide antagonist of C/EBPβ and the composition comprising an immunomodulator can be the same composition or can be different compositions. If the peptide antagonist and the immunomodulating agent are comprised in two separate compositions, the two compositions can be administered to the subject at the same time or at different times, including on different days.

Each of the composition comprising a peptide antagonist and the composition comprising an immunomodulator is administered to the subject more than once. In one embodiment, administration of the peptide antagonist of C/EBPβ can occur once weekly for a duration of at least three weeks *(i.e.,* three administrations), six weeks *(i.e.,* six administrations), nine weeks, twelve weeks, three months, six months, nine months, or twelve months. In another embodiment, administration can occur once every two weeks for a duration of at least four weeks *(i.e.,* two administrations), eight weeks *(i.e.,* four administrations), twelve weeks, three months, six months, nine months, or twelve months. In some embodiments, a patient can be administered the peptide antagonist of C/EBPβ once weekly for a duration of at least three weeks, six weeks, nine weeks, twelve weeks, three months, six months, nine months, or twelve months, followed by administration once every two weeks for at least four weeks, eight weeks, twelve weeks, three months, six months, nine months, or twelve months.

In one embodiment, administration of the immunomodulator can occur once weekly for a duration of at least three weeks *(i.e.,* three administrations), six weeks (*i.e.*, six administrations), nine weeks, twelve weeks, three months, six months, nine months, or twelve months. In another embodiment, administration can occur once every two weeks for a duration of at least four weeks *(i.e.,* two administrations), eight weeks *(i.e.,* four administrations), twelve weeks, three months, six months, nine months, or twelve months. In a further embodiment, administration of the immunomodulator can occur once every three weeks for a duration of at least six weeks *(i.e.,* two administrations), nine weeks *(i.e.,* three administrations), twelve weeks, three months, six months, nine months, or twelve months. In an additional embodiment, administration of the immunomodulator can occur once every four weeks for a duration of at least eight weeks *(i.e.,* two administrations), twelve weeks *(i.e.,* three administrations), three months, six months, nine months, or twelve months. Likewise, administration of the immunomodulator can occur once a month for a duration of at least two months *(i.e.,* two administrations), three months *(i.e.,* three administrations), six months, nine months, or twelve months.

For purposes of the present disclosure, "combination therapy" means that the treatment period comprising administration of the peptide antagonist of C/EBPβ overlaps with the treatment period comprising administration of the immunomodulator.

### III. Methods of Use

Subjects in need of the methods of the invention are patients diagnosed with a solid tumor. For example, the subject can have a locally advanced solid tumor or a metastatic inoperable tumor. In some embodiments, the subject has basal cell carcinoma, bladder cancer, cervical cancer, cholangiocarcinoma, colorectal cancer, endometrial cancer, esophageal cancer, gastric cancer, hepatocellular carcinoma, head and neck cancer, Merkel cell carcinoma, melanoma, renal cell carcinoma, squamous cell carcinoma, triple-negative breast cancer, or urothelial cell carcinoma.

In particular embodiments, the subject has a melanoma, carcinoma, or sarcoma. In one embodiment, the melanoma is a cutaneous melanoma or a mucosal melanoma. In one embodiment, the carcinoma is adenocarcinoma, such as bladder adenocarcinoma, colorectal adenocarcinoma, pancreatic adenocarcinoma, gastric/signet ring adenocarcinoma, or small bowel adenocarcinoma. In one embodiment, the sarcoma is abdominal sarcoma or myofibroblastic sarcoma. In particular embodiments of the invention, combination therapy with a peptide antagonist of C/EBPβ, such as ST101, and an immunomodulator can inhibit tumor growth, reduce tumor volume, or a combination thereof.

Efficacy of treatment can be evaluated by one or more known measures. For example, patients subjected to methods of the invention can experience outcomes including extended survival, improved progression-free survival, improved duration of response, longer remission, reduced risk of relapse, and/or improved tumor response to treatment with the combination therapy of the invention, compared with the same outcome(s) in patients not subjected to methods of the invention, *i.e.,* control patients. An outcome in a patient treated by a method of the invention can be compared, for example, to the median outcome in a population of control patients. The population of control patients can be administered, for example, a regimen selected from the group consisting of a placebo, surgery, radiation, chemotherapy, immunotherapy, hormone-based therapy, or targeted therapy. In another embodiment, a patient subjected to the combination therapy of the invention can be compared to a population of control patients administered treatment with only one of a peptide antagonist of C/EBPβ or an immunomodulator. Comparisons can be analyzed statistically using, for example, the Wilcoxon signed rank test or the Kaplan-Meier method.

Response to treatment compares one or more measures of efficacy after a treatment regimen, as compared to baseline, e.g., prior to treatment with combination therapy. A baseline assessment is preferably performed within 24, 48, or 72 hours, or within 1, 2, 3, or 4 weeks prior to the first treatment. In a one preferred embodiment, a baseline assessment is performed within 24 hours prior to the first treatment.

"Tumor burden" is the total mass or total size of cancerous tissue in a patient's body. Tumor response can be evaluated by measures including objective response rate, disease control rate, and duration of response. These parameters can be determined, for example, by revised Response Evaluation Criteria in Solid Tumors (RECIST 1.1) (Eisenhauer 2009), by modified response assessment in neuro-oncology (mRANO) (Ellingson 2017), or by PCWG3 guidelines (Scher 2016), depending on the type of tumor.

Objective response rate assesses reduction of tumor size, for example, tumor diameter, which can be determined by clinical examination and/or imaging. Where a patient has multiple tumors, tumor size can optionally be expressed as the average diameter of all tumors or by the sum of diameters of all tumors. Superficial tumors can be measured clinically, for instance, using calipers or by photography and ruler measurement. Imaging methods include computed tomography (CT), typically with contrast; X-ray; magnetic resonance imaging (MRI): and positron emission tomography (PET), such as (18)F-fluorodeoxyglucose PET. In one preferred embodiment, CT is utilized to assess tumor response, for example, in a LA/MBC patient or a melanoma patient. In another preferred embodiment, MRI, such as gadolinium-enhanced MRI, is utilized to assess tumor response, for example, in a GBM patient. Accordingly, in one aspect, the invention provides a method of reducing tumor burden, *i.e.,* tumor mass and/or tumor size, in a patient, the method comprising administering to the patient combination therapy comprising a peptide antagonist of C/EBPβ, such as ST101, and an immunomodulator. Reduction in tumor burden is measured relative to baseline.

In certain embodiments, particularly those in which assessment is by RECIST 1.1, disease control rate defines the level of tumor response as complete response (CR), which is the disappearance of tumor(s); partial response (PR), which is a decrease of at least 30%, in the size of tumor(s); stable disease, in which there is no change in the size of tumor(s); or disease progression, which is an increase of at least 20%, in tumor size and/or new lesions.

Duration of response is the length of time from the achievement of a response until disease progression, *i.e.,* the period in which a tumor does not grow or spread, or death. Duration of response in patients receiving combination therapy of the invention can be, for example, at least 4, 6, 8, 10, or 12 weeks, at least 4, 6, 8, 10, 12, 16, 18, or 24 months, or at least 3, 4. or 5 years. Accordingly, in one aspect, the invention provides a method of increasing the duration of response in a patient, the method comprising administering to the patient combination therapy comprising a peptide antagonist of C/EBPβ and an immunomodulator. Increase in duration of response is measured relative to the median duration of response in a control population.

Survival can be assessed as overall survival, *i.e.,* the length of time a patient lives, or as progression-free survival, *i.e.,* the length of time a patient is treated without progression or worsening of the disease. Survival can be measured from the date of diagnosis or from the date that treatment commences. Overall survival, median overall survival, progression-free survival, and median progression-free survival can be calculated, for example, by Kaplan-Meier analysis, based on the response to treatment. Accordingly, in one aspect, the invention provides a method of increasing overall survival in a patient, the method comprising administering to the patient combination therapy comprising a peptide antagonist of C/EBPβ and an immunomodulator. Increase in overall survival is measured relative to the median overall survival in a control population. In another aspect, the invention provides a method of increasing progression-free survival in a patient, the method comprising administering to the patient combination therapy comprising a peptide antagonist of C/EBPβ and an immunomodulator. Increase in progression-free survival is measured relative to the median progression-free survival in a control population.

A patient is successfully treated according to the methods of the invention if the patient experiences or displays at least one of the following outcomes after administration of combination therapy:
- undetectability of the tumor (or at least one tumor, if multiple tumors are present at baseline);
- at least about 10%, 20%, 30%, 40%, 50%, 60%, 70%, 80%, or 90% reduction in tumor size compared to baseline;
- no significant increase (e.g., less than 20%) in tumor size compared to baseline:
- significantly increased duration of response, optionally compared with median duration of response of a population of control patients;
- significantly increased progression-free survival, optionally compared with median progression-free survival of a population of control patients;
- significantly increased overall survival, optionally compared with median overall survival of a population of control patients.

### EXAMPLES

Embodiments of the present disclosure can be further defined by reference to the following non-limiting examples. It will be apparent to those skilled in the art that many modifications, both to materials and methods, can be practiced without departing from the scope of the present disclosure.

### Example 1. Antagonist of C/EBPβ Reprograms Myeloid-Derived Suppressor Cell Polarization and Decreases Tumor-Associated Tregs

Myeloid-derived suppressor cells (MDSCs) play a role in cancer progression and other associated diseases by suppressing both innate and adaptive immune response. Depletion of MDSCs, for example, by conditional depletion of C/EBPβ in hematopoietic lineage cells, inhibits MDSC immunosuppressive activity and leads to markedly enhanced anti-tumor immunity.

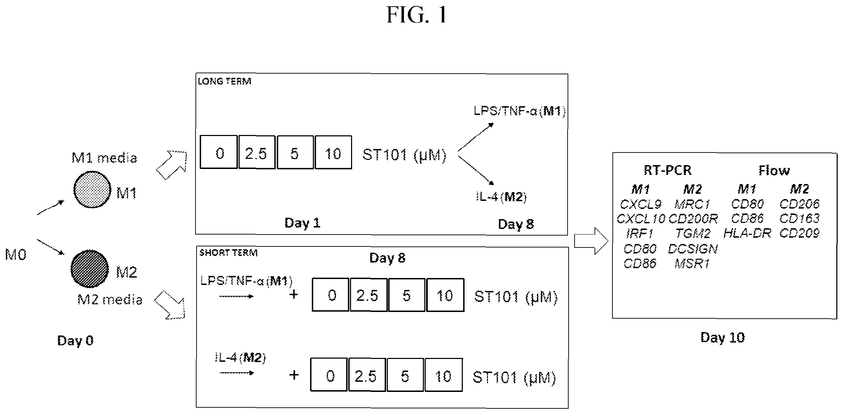

Primary human macrophages were cultured from peripheral blood mononuclear cells (hPBMCs) and activated toward the M1 or M2 phenotype by lipopolysaccharide (LPS) and TNFα (M1) or IL-4 (M2), respectively, in the presence of the C/EBPβ antagonist, ST101 (FIG. 1). Macrophage M1 (CD80, CD86) and M2 (CD163, CD206) expression were analyzed by flow cytometry and rtPCR (FIG. 1). Paired biopsy tissue from the ST101 Phase 1-2 clinical study in patients with advanced unresectable and metastatic solid tumors were collected during screening (prior to ST101 exposure) and within 24 hours of ST101 administration during cycle 2 of therapy. Nanostring gene expression analysis was performed to determine differential gene expression and the impact of ST101 on the tumor microenvironment.

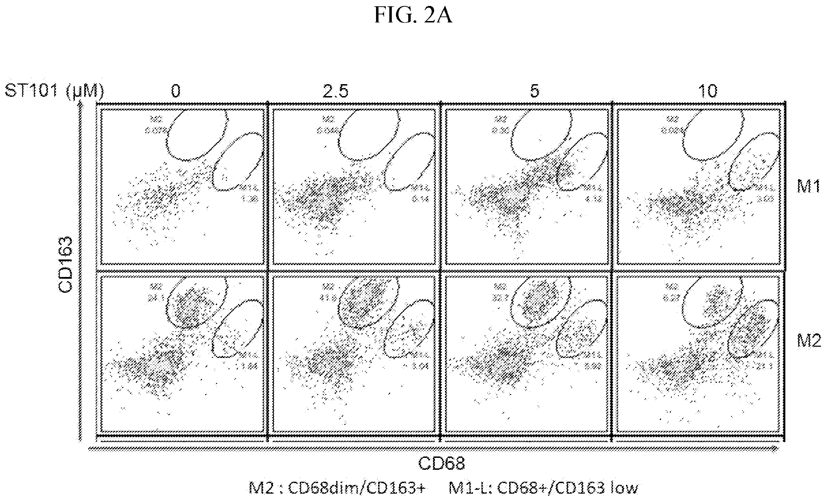

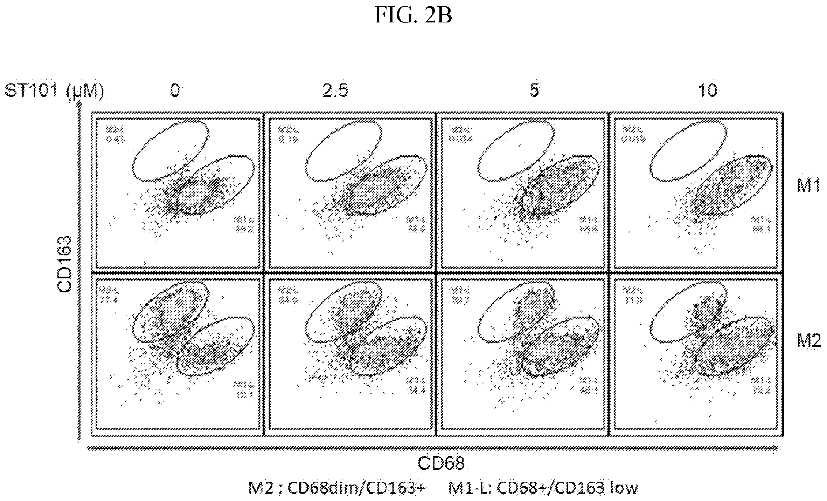

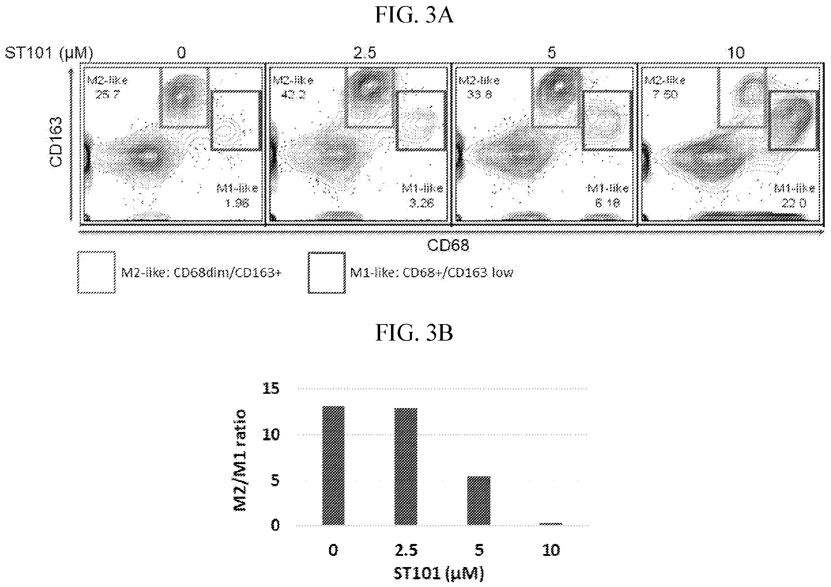

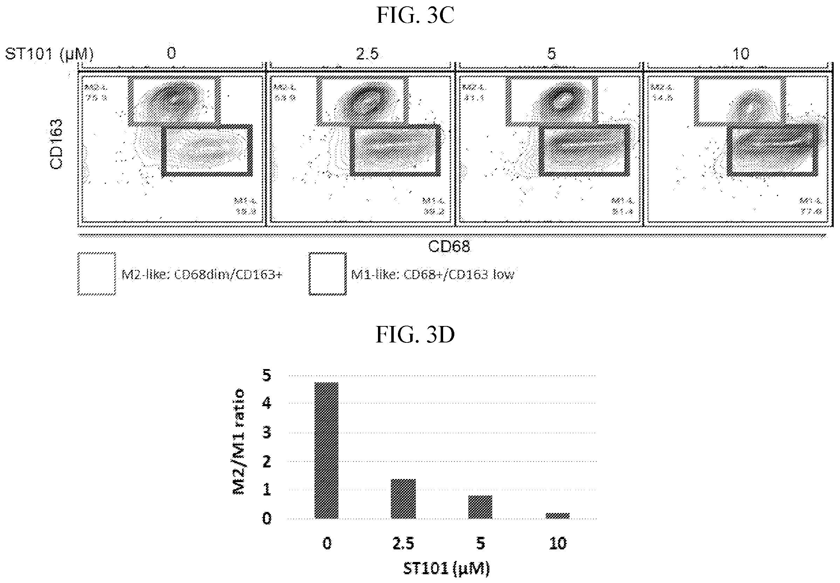

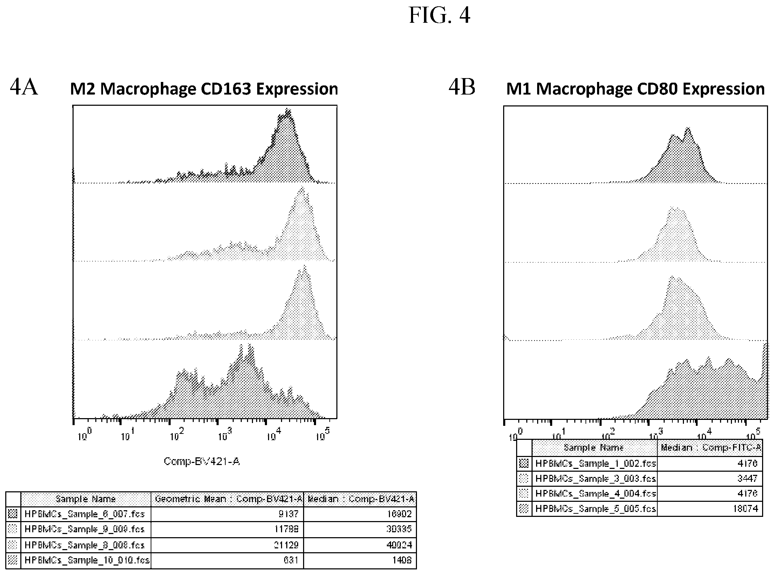

Treatment with pharmacologically relevant concentrations of ST101 (2.5, 5 or 10 µM) resulted in dose-dependent reduction in M2+ macrophage and corresponding induction of M1+ macrophage **(****FIG 2A****,** **2B****;** **FIG. 3A****,** **3C****;** **FIG. 4A-4B****).** At the highest ST101 concentration, a 12-fold reduction in the M2 to M1 ratio was observed without substantial impact on cell viability **(****FIG. 3B****,** **3D****).**

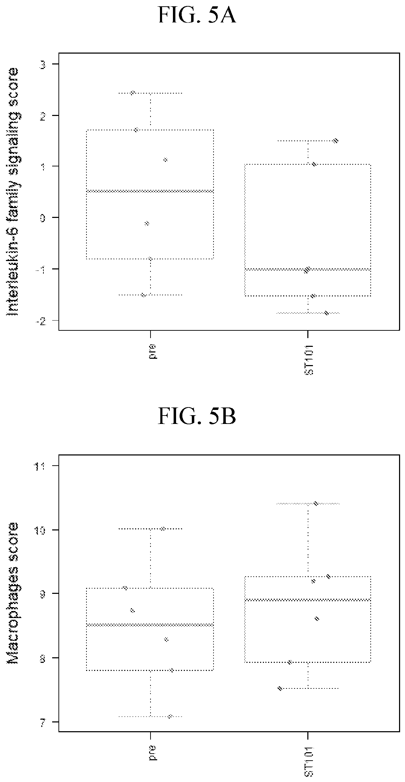

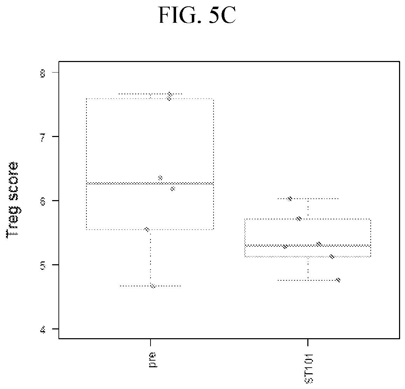

Paired patient biopsy tissue from the ST101 Phase 1-2 clinical study indicates a decrease in C/EBPβ target gene IL-6 signaling, an important driver of the M2 macrophage phenotype. Decreased IL-6 signaling resulted in an increase in the tumor-infiltrating macrophage vs. tumor-infiltrating lymphocyte (TIL) ratio, and a decrease in the regulatory T cell (Treg) vs. TIL ratio in tumor samples of treated patients **(****FIG. 5A-****5C).**

Overall, these results validate the potential of ST101 in reprogramming M2 macrophages to pro-inflammatory M1 macrophages, support a novel, macrophage-driven mechanism of action for ST101 as an anticancer agent and support further exploration of ST101 in immune-oncology therapeutic strategies. Importantly, the impact of ST1 01 on macrophage polarization is likely to act in concert with direct cytotoxicity of C/EBPβ-driven cancers, and raises the possibility ST101 target population may extend beyond the cancer types driven by C/EBPβ.

### Example 2. Antagonist of C/EBPβ Potently Activated CD8+ T-Cells

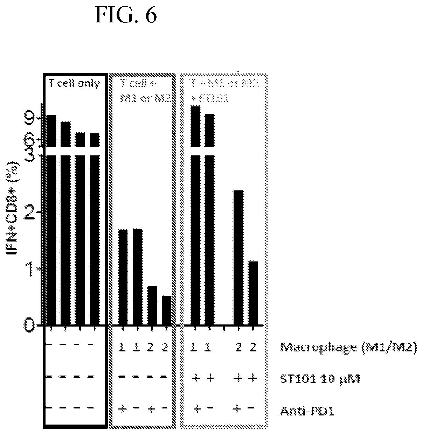

Cultured human T cells (matched donor) were co-incubated with M1 or M2 macrophage culture. T cell activation was measured in CD8+ cells by intracellular interferon-gamma staining. In the presence of M2 macrophages, T cell activation was suppressed, as indicated by reduced percentage of interferon-gamma positive cells. Addition of ST101 to a culture of matched donor human T cells with M2 macrophages restored T cell activity. Treatment with ST101 of a culture of matched donor human T cells with M1 macrophages increased activity relative to an untreated culture of matched donor human T cells with M1 macrophages. Results are shown in **FIG. 6****.**

### Example 3. C/EBPβ Antagonist and Checkpoint Inhibitor Show Anti-Tumor Activity

Using a mouse model of triple-negative breast cancer (TNBC), we show that inhibition of C/EBPβ and PD-1 results in enhanced anti-tumor activity, compared with either treatment alone. Briefly, 4T1-luc TNBC tumor cells were implanted orthotopically into the mammary fatpad of immune-competent Balb/c mice. Tumors reached approximately 100 mm³ on day 10 post-implantation, when treatment was initiated. Animals received 10 mg/kg ST101 subcutaneously on days 10, 12, 14, 16, 18, and 22 post-tumor inoculation and/or 12.5 mg/kg ant-PD-1 antibody (BioXCell, Lebanon, NH) intraperitoneally on days 10, 14, and 22 post-tumor inoculation. Results are shown in **FIG. 7****.**

### REFERENCES

Angelastro JM, et al. Selective destruction of glioblastoma cells by interference with the activity or expression of ATF5. Oncogene 2006; 25: 907-916.
Bernal F, et al. Reactivation of the p53 Tumor Suppressor Pathway by a Stapled p53 Peptide. J. Am. Chem. Soc. 2007: 129:2456-2457.
Bezy O, et al. Delta-interacting protein A, a new inhibitory partner of CCAAT/enhancer-binding protein beta, implicated in adipocyte differentiation. J Biol Chem 2005; 280: 11432-11438.
Bird GH, et al. Biophysical Determinants for Cellular Uptake of Hydrocarbon-Stapled Peptide Helices. Nat. Chem. Biol. 2017; 12:845-852.
Cates CC, et al. Regression/eradication of gliomas in mice by a systemically-deliverable ATF5 dominant-negative peptide. Oncotarget 2016; 7: 12718-12730.
Dougherty PG, et al. Understanding Cell Penetration of Cyclic Peptides. Chem. Rev. 2019: 119(17):10241-10287.
Eisenhauer EA, et al. New response evaluation criteria in solid tumours: Revised RECIST guideline (version 1.1). Eur J Cancer 2009: 45(2):228-247.
Ellingson B, et al. Modified Criteria for Radiographic Response Assessment in Glioblastoma Clinical Trials. Neurotherapeutics. 2017; 14:307-320.
Homma J, et al. Increased expression of CCAAT/enhancer binding protein beta correlates with prognosis in glioma patients. Oncol Rep 2006: 15: 595-601.
Huggins CJ, et al. C/EBPgamma suppresses senescence and inflammatory gene expression by heterodimerizing with C/EBPbeta. Mol Cell Biol 2013; 33: 3242-3258.
Karpel-Massler G, et al. A Synthetic Cell-Penetrating Dominant-Negative ATF5 Peptide Exerts Anticancer Activity against a Broad Spectrum of Treatment-Resistant Cancers. Clin Cancer Res 2016; 22: 4698-4711.
Marigo I, et al. Tumor-induced tolerance and immune suppression depend on the C/EBPβ transcription factor. Immunity. 2010; 32:790-802.
Ruffell D, et al. CREB-C/EBPβ cascade induces M2 macrophage-specific gene expression and promotes muscle injury repair. PNAS. 2009; 106:17475-17480.
Scher HI, et al. Trial Design and Objectives for Castration-Resistant Prostate Cancer: Updated Recommendations from the Prostate Cancer Clinical Trials Working Group 3. J Clin Oncology. 2016:34(12):1402-1418.
Sheng Z, et al. An activating transcription factor 5-mediated survival pathway as a target for cancer therapy? Oncotarget 2010; 1: 457-460.
Zhang ZY, et al. Stabilization of ATF5 by TAK1-Nemo-like kinase critically regulates the interleukin-1beta-stimulated C/EBP signaling pathway. Mol Cell Biol 2015: 35: 778-788.
Zhao Y, et al. p300-dependent acetylation of activating transcription factor 5 enhances C/EBPbeta transactivation of C/EBPalpha during 3T3-L1 differentiation. Mol Cell Biol 2014; 34: 315-324.

The present invention is further described by the following items.
1. A method of inhibiting growth of a solid tumor in a subject, the method comprising combination therapy with: (i) a pharmaceutical composition comprising an effective amount of a peptide antagonist of CCAAT-enhancer-binding protein β (C/EBPβ) and (ii) a pharmaceutical composition comprising an effective amount of an immunomodulator; wherein the pharmaceutical composition comprising the antagonist of C/EBPβ and the pharmaceutical composition comprising the immunomodulator are administered to the subject together or separately.
2. A method of reducing solid tumor volume in a subject, the method comprising combination therapy with: (i) a pharmaceutical composition comprising an effective amount of a peptide antagonist of CCAAT-enhancer-binding protein β (C/EBPβ) and (ii) a pharmaceutical composition comprising an effective amount of an immunomodulator: wherein the pharmaceutical composition comprising the antagonist of C/EBPβ and the pharmaceutical composition comprising the immunomodulator are administered to the subject together or separately.
3. A method of treating a solid tumor in a in a subject, the method comprising combination therapy with: (i) a pharmaceutical composition comprising an effective amount of a peptide antagonist of CCAAT-enhancer-binding protein β (C/EBPβ) and (ii) a pharmaceutical composition comprising an effective amount of an immunomodulator; wherein the pharmaceutical composition comprising the antagonist of C/EBPβ and the pharmaceutical composition comprising the immunomodulator are administered to the subject together or separately.
4. The method of any preceding item, wherein the solid tumor is a melanoma, a carcinoma, or a sarcoma.
5. The method of any preceding item, wherein the subject has been diagnosed with locally advanced or metastatic breast cancer (LA/MBC), melanoma, glioblastoma (GBM), or castration-resistant prostate cancer (CRPC).
6. The method of any one of items 1 to 3, wherein the peptide antagonist comprises the D-amino acid sequence VAEAREELERLEARLGQARGEL (SEQ ID NO: 1).
7. The method of any one of items 1 to 3, wherein the peptide antagonist comprises the amino acid sequence LEGRAQGLRAELRELEERAEAV (SEQ ID NO: 3).
8. The method of any preceding item, wherein the peptide antagonist is a cell-penetrating peptide.
9. The method of any one of items 1 to 3, wherein the peptide antagonist is ST101.
10. The method of item 9, wherein the peptide antagonist is administered to the subject at a dose of about 0.5-16 mg/kg.
11. The method of item 9, wherein the peptide antagonist is administered to the subject at a dose of about 500 mg.
12. The method of any one of items 1 to 3, wherein the immunomodulator is selected from the group consisting of a checkpoint inhibitor, a cytokine, an immune adjuvant.
13. The method of any one of items 1 to 3, wherein the immunomodulator is an antibody or an antibody-drug conjugate.
14. The method of item 13, wherein the antibody or antibody-drug conjugate is selected from the group consisting of amivantamab, belantamab mafodotin-blmf, bevacizumab, cetuximab, denosumab, dinutuximab, enfortumab vedotin-ejfv, margetuximab, naxitamab-gqgk, necitumumab, panitumumab, pertuzumab, ramucirumab, Sacituzumab govitecan-hziy, tebentafusp-tebn, tisotumab vedotin, trastuzumab, trastuzumab deruxtecan, and trastuzumab emtansine.
15. The method of item 13, wherein the antibody is an anti-PD-1 antibody.
16. The method of item 12, wherein the checkpoint inhibitor targets at least one of PD-1, PD-L1, CTLA-4, or LAG-3.
17. The method of item 12, wherein the checkpoint inhibitor is selected from the group consisting of atezolizumab, avelumab, cemiplimab, dostarlimab, durvalumab, ipilimumab, nivolumab, pembrolizumab, relatimab, and a combination thereof.
18. The method of item 12, wherein the cytokine targets at least one of the IL-2 pathway, the IL-2R pathway, the IFNAR1 pathway, or the IFNAR2 pathway.
19. The method of item 12, wherein the cytokine is selected from the group consisting of aldesleukin, granulocyte-macrophage colony-stimulating factor, interferon alfa-2a, interferon alfa-2b, and peginterferon alfa-2b.
20. The method of item 12, wherein the immune adjuvant targets the Toll-like receptor 7 pathway or the Toll-like receptor 3 pathway.
21. The method of item 12, wherein the immune adjuvant is selected from the group consisting of Imiquimod and Poly ICLC.
22. The method of any preceding item, wherein the pharmaceutical composition comprising the antagonist of C/EBPβ and/or the pharmaceutical composition comprising the immunomodulator is administered intravenously.
23. The method of any preceding item, wherein the pharmaceutical composition comprising the antagonist of C/EBPβ and the pharmaceutical composition comprising the immunomodulator are administered to the subject on different days.
24. The method of any one of items 1 to 23, wherein the pharmaceutical composition comprising the antagonist of C/EBPβ is administered once weekly for at least three weeks.
25. The method of any one of items 1 to 23, wherein the pharmaceutical composition comprising the antagonist of C/EBPβ is administered once every two weeks for at least four weeks.
26. The method of any one of items 1 to 25, wherein the pharmaceutical composition comprising the immunomodulator is administered once weekly for at least three weeks.
27. The method of any one of items 1 to 25, wherein the pharmaceutical composition comprising the immunomodulator is administered once every two weeks for at least four weeks.
28. The method of any one of items 1 to 25, wherein the pharmaceutical composition comprising the immunomodulator is administered once every three weeks for at least six weeks.
29. A pharmaceutical composition comprising an effective amount of a peptide antagonist of CCAAT-enhancer-binding protein β (C/EBPβ) and a pharmaceutical composition comprising an effective amount of an immunomodulator for use in a method of combination therapy for inhibiting growth of a solid tumor in a subject.
30. A pharmaceutical composition comprising an effective amount of a peptide antagonist of CCAAT-enhancer-binding protein β (C/EBPβ) and a pharmaceutical composition comprising an effective amount of an immunomodulator for use in a method of combination therapy for reducing solid tumor volume in a subject.
31. A pharmaceutical composition comprising an effective amount of a peptide antagonist of CCAAT-enhancer-binding protein β (C/EBPβ) and a pharmaceutical composition comprising an effective amount of an immunomodulator for use in a method of combination therapy for treating a solid tumor in a human subject.
32. The pharmaceutical compositions of any one of items 29 to 31. wherein the antagonist of C/EBPβ is ST101, and wherein the immunomodulator is a PD-1 inhibitor.

## Claims

1. A pharmaceutical composition comprising a peptide antagonist of CCAAT-enhancer-binding protein β (C/EBPβ), wherein the peptide antagonist comprises the D-amino acid sequence VAEAREELERLEARLGQARGELKKWKMRRNQFWLKLQR (SEQ ID NO: 2); and
a pharmaceutical composition comprising an immunomodulator; wherein the immunomodulator is a PD-1 inhibitor,
for use in combination therapy for treating a solid tumor in a subject, wherein the peptide antagonist is to be administered to the subject via intravenous infusion at a dose of about 0.5-16 mg/kg; and wherein the pharmaceutical composition comprising the antagonist of C/EBPβ and the pharmaceutical composition comprising the immunomodulator are to be administered to the subject together or separately.

2. The pharmaceutical compositions for the use according to claim 1, wherein the solid tumor is a melanoma, a carcinoma, or a sarcoma.

3. The pharmaceutical compositions for the use according to claim 1, wherein the subject has been diagnosed with locally advanced or metastatic breast cancer (LA/MBC), melanoma, glioblastoma (GBM), or castration-resistant prostate cancer (CRPC).

4. The pharmaceutical compositions for the use according to any one of claims 1 to 3, wherein the peptide antagonist is administered to the subject at a dose of about 500 mg.

5. The pharmaceutical compositions for the use according to any one of claims 1 to 4, wherein the immunomodulator is an anti-PD-1 antibody.

6. The pharmaceutical compositions for the use according to any one of claims 1 to 4, wherein the immunomodulator is a checkpoint inhibitor selected from the group consisting of atezolizumab, avelumab, cemiplimab, dostarlimab, durvalumab, nivolumab, pembrolizumab, and a combination thereof.

7. The pharmaceutical compositions for the use according to any one of claims 1 to 6, wherein the pharmaceutical composition comprising the antagonist of C/EBPβ and the pharmaceutical composition comprising the immunomodulator are administered to the subject on different days.

8. The pharmaceutical compositions for the use according to any one of claims 1 to 7, wherein the pharmaceutical composition comprising the antagonist of C/EBPβ is administered once weekly for at least three weeks.

9. The pharmaceutical compositions for the use according to any one of claims 1 to 7, wherein the pharmaceutical composition comprising the antagonist of C/EBPβ is administered once every two weeks for at least four weeks.

10. The pharmaceutical compositions for the use according to any one of claims 1 to 9, wherein the pharmaceutical composition comprising the immunomodulator is administered once weekly for at least three weeks.

11. The pharmaceutical compositions for the use according to any one of claims 1 to 9, wherein the pharmaceutical composition comprising the immunomodulator is administered once every two weeks for at least four weeks.

12. The pharmaceutical compositions for the use according to any one of claims 1 to 9, wherein the pharmaceutical composition comprising the immunomodulator is administered once every three weeks for at least six weeks.
